# EUROPÄISCHE PATENTSCHRIFT

(11) **EP 1 763 501 B1**
(45) Veröffentlichungstag und Bekanntmachung des Hinweises auf die Patenterteilung: **10.06.2009**
(21) Anmeldenummer: 05769990.2
(22) Anmeldetag: 22.06.2005
(51) Int. Cl.: C07C 211/43, G03G 5/147, C09K 11/06

(54) **VERBINDUNGEN FÜR ORGANISCHE ELEKTRONISCHE VORRICHTUNGEN**
COMPOUNDS FOR ORGANIC ELECTRONIC DEVICES
COMPOSES POUR DISPOSITIFS ELECTRONIQUES ORGANIQUES

(30) Priorität: 26.06.2004 DE 102004031000; 31.01.2005 EP 05001891
(43) Veröffentlichungstag der Anmeldung: 21.03.2007
(73) Patentinhaber: Merck Patent GmbH, 64293 Darmstadt (DE)
(72) Erfinder: HEIL, Holger, 64285 Darmstadt (DE); STÖSSEL, Philipp, 60487 Frankfurt (DE); VESTWEBER, Horst, 34630 Gilserberg (DE)
(86) Internationale Anmeldenummer: PCT/EP2005/006728
(87) Internationale Veröffentlichungsnummer: WO 2006/000389

(56) Entgegenhaltungen:
- EP-A- 1 074 601
- EP-A- 1 291 723
- US-A- 4 769 302
- US-A1- 2002 058 156

## Beschreibung

Die vorliegende Erfindung beschreibt neue Verbindungen und deren Einsatz in organischen Elektrolumineszenzvorrichtungen.

Der Einsatz halbleitender organischer Verbindungen, die zur Emission von Licht im sichtbaren Spektralbereich befähigt sind, in organischen Elektrolumineszenzvorrichtungen (OLEDs) steht gerade am Anfang der Markteinführung. Der allgemeine Aufbau derartiger Vorrichtungen ist beispielsweise in US 4539507, US 5151629, EP 0676461 und WO 98/27136 beschrieben. Für einfache OLEDs enthaltende Vorrichtungen ist die Markteinführung bereits erfolgt, wie die Autoradios der Firma Pioneer, die Mobiltelefone der Firmen Pioneer und SNMD oder eine Digitalkamera der Firma Kodak mit "organischem Display" belegen. Weitere derartige Produkte stehen kurz vor der Einführung.

Allerdings zeigen diese Vorrichtungen immer noch erhebliche Probleme, die einer dringenden Verbesserung bedürfen:
1. Die Effizienz ist gerade bei fluoreszierenden OLEDs immer noch zu niedrig und muss verbessert werden.
2. Die operative Lebensdauer ist insbesondere bei blauer Emission immer noch gering, so dass bis dato nur einfache Anwendungen kommerziell realisiert werden konnten.
3. Die Betriebsspannung ist gerade bei fluoreszierenden OLEDs recht hoch und sollte daher weiter verringert werden, um die Leistungseffizienz zu verbessern. Das ist insbesondere für mobile Anwendungen von großer Bedeutung.
4. Viele blau emittierende Emitter, die sowohl aromatische Amine, wie auch Doppelbindungssysteme enthalten, sind thermisch nicht stabil und zersetzen sich beim Sublimieren oder beim Aufdampfen. Dadurch ist die Verwendung dieser Systeme nicht bzw. nur unter großen Verlusten und mit hohem technischen Aufwand möglich.

Als nächstliegender Stand der Technik kann die Verwendung bestimmter Arylvinylamine von Idemitsu genannt werden (z. B. WO 04/013073, WO 04/016575, WO 04/018587). Darin werden sehr gute Lebensdauern bei tiefblauer Emission zitiert. Allerdings sind diese Ergebnisse, wie man sieht, stark abhängig vom verwendeten Hostmaterial, so dass die zitierten Lebensdauern nicht als Absolutwerte verglichen werden können, sondern immer nur bei Einsatz in einem optimierten System. Weiterhin sind diese Verbindungen thermisch instabil und lassen sich nicht unzersetzt verdampfen, was einen hohen technischen Aufwand für die Aufdampfung erfordert und somit einen deutlichen technischen Nachteil darstellt. Ein weiterer Nachteil ist die Emissionsfarbe dieser Verbindungen. Während Idemitsu tiefblaue Emission (CIE-y-Koordinaten im Bereich von 0.15-0.18) zitiert, konnten diese Farbkoordinaten nicht in einfachen Vorrichtungen gemäß dem Stand der Technik reproduziert werden. Im Gegenteil erhält man hier grünblaue Emission. Es ist nicht offensichtlich, wie mit diesen Verbindungen tatsächlich blaue Emission erzeugt werden kann.

Aus EP 1291723 sind weiterhin verschiedene aromatische Amine und Stilbenamine als Photorezeptoren für die Elektrophotographie bekannt. Hierin wird neben einer Vielzahl weiterer Verbindungen auch ein Tris(biphenyl)amin-Derivat offenbart, welche mit Diethylamino-substituierten Styrylgruppen substituiert ist.

Es besteht also weiterhin Bedarf an blau emittierenden Verbindungen, die in organischen Elektrolumineszenzvorrichtungen zu guten Effizienzen und gleichzeitig zu hohen Lebensdauern führen und die technisch unproblematisch zu verarbeiten sind. Es wurde nun überraschend gefunden, dass organische Elektrolumineszenzvorrichtungen, die bestimmte - im Folgenden aufgeführte - Verbindungen als blau emittierende Dotanden in einem Hostmaterial enthalten, deutliche Verbesserungen gegenüber dem Stand der Technik aufweisen, Mit diesen Materialien ist es möglich, längere Lebensdauern bei höherer Effizienz zu erhalten. Außerdem lassen sich diese Verbindungen im Gegensatz zu Materialien gemäß dem Stand der Technik ohne merkliche Zersetzung sublimieren und aufdampfen und sind daher deutlich leichter zu handhaben als Materialien gemäß dem Stand der Technik. Diese Verbindungen und deren Verwendung in OLEDs sind daher Gegenstand der vorliegenden Erfindung.

Gegenstand der Erfindung sind Verbindungen gemäß Formel (1), 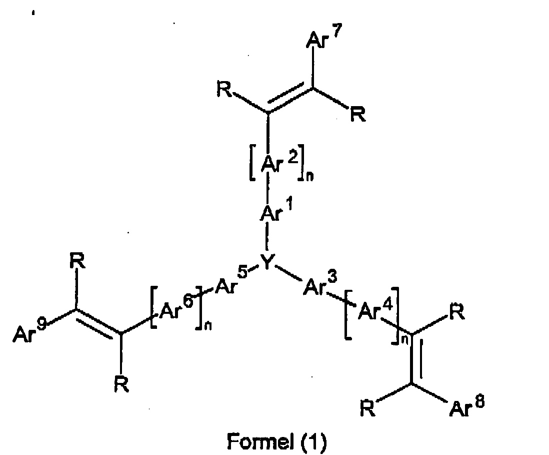 wobei für die verwendeten Symbole und Indizes gilt:
- Y: ist bei jedem Auftreten Stickstoff, Phosphor, Arsen, Antimon, P=O, As=O oder Sb=O;
- Ar¹, Ar², Ar³, Ar⁴, Ar⁵, Ar⁶: ist bei jedem Auftreten gleich oder verschieden eine bivalente Aryl- oder Heteroarylgruppe mit 2 bis 24 C-Atomen, die mit einem oder mehreren Resten R¹ substituiert sein kann;
- Ar⁷, Ar⁸, Ar⁹: ist bei jedem Auftreten gleich oder verschieden eine monovalente Aryl- oder Heteroarylgruppe mit 2 bis 24 C-Atomen, die mit einem oder mehreren Resten R¹ substituiert sein kann;
- R: ist bei jedem Auftreten gleich oder verschieden H, CN, eine geradkettige, verzweigte oder cyclische Alkylgruppe mit 1 bis 40 C-Atomen, die mit einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können, oder eine monovalente Aryl- oder Heteroarylgruppe mit 2 bis 24 C-Atomen, die mit einem oder mehreren Resten R¹ substituiert sein kann;
- R¹: ist bei jedem Auftreten gleich oder verschieden H, F, Cl, Br, I, CN, NO₂, eine geradkettige, verzweigte oder cyclische Alkyl-, Alkoxy- oder Thioalkoxygruppe mit 1 bis 40 C-Atomen, die mit einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können, oder eine Aryl- oder Heteroarylgruppe mit 2 bis 24 C-Atomen, welche durch einen oder mehrere Reste R² substituiert sein kann, oder eine Aryloxy- oder Heteroaryloxygruppe mit 2 bis 24 aromatischen C-Atomen, die durch einen oder mehrere Reste R² substituiert sein kann, oder eine Kombination aus zwei, drei, vier oder fünf dieser Systeme; dabei können auch zwei oder mehrere Substituenten R¹ sowohl am selben Ring wie auch an unterschiedlichen Ringen miteinander ein mono- oder polycyclisches, aliphatisches oder aromatisches Ringsystem bilden;
- R²: ist bei jedem Auftreten gleich oder verschieden H oder ein aliphatischer oder aromatischer Kohlenwasserstoffrest mit 1 bis 20 C-Atomen;
- n: ist bei jedem Auftreten gleich oder verschieden 1, 2, 3, 4 oder 5.

Unter einer Arylgruppe bzw. einer Heteroarylgruppe im Sinne dieser Erfindung wird eine aromatische Gruppe bzw. heteroaromatische Gruppe mit einem gemeinsamen aromatischen Elektronensystem verstanden. Dies kann im Sinne dieser Erfindung ein einfacher Homo- oder Heterocyclus sein, beispielsweise Benzol, Pyridin, Thiophen, etc., oder es kann ein kondensiertes aromatisches Ringsystem sein, in dem mindestens zwei aromatische oder heteroaromatische Ringe, beispielsweise Benzolringe, miteinander "verschmolzen", d. h. durch Anellierung einander ankondensiert sind, also mindestens eine gemeinsame Kante und dadurch auch ein gemeinsames aromatisches System aufweisen. So sind beispielsweise Systeme wie Naphthalin, Anthracen, Phenanthren, Pyren, etc. als Arylgruppen und Chinolin, Acridin, Benzothiophen, Carbazol, etc. als Heteroarylgruppen im Sinne dieser Erfindung zu sehen, während beispielsweise Biphenyl, Fluoren, Spirobifluoren, etc. keine Arylgruppen darstellen, da es sich hierbei um separate aromatische Elektronensysteme handelt.

Im Rahmen der vorliegenden Erfindung werden unter einer C₁- bis C₄₀-Alkylgruppe besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einer C₂-C₂₄ Aryl- oder Heteroarylgruppe, die je nach Verwendung monovalent oder bivalent sein kann, die noch jeweils mit den oben genannten Resten R¹ substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Chrysen, Perylen, Fluoranthen, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.
Die durch Kombination dieser Systeme und Bildung zusätzlicher Ringsysteme entstehenden Systeme sind bevorzugt Biphenylen, Terphenylen, Fluoren, Spirobifluoren, Dihydrophenanthren, Dihydropyren, Tetrahydropyren oder cis- oder trans-Indenofluoren.

Bevorzugt sind Verbindungen gemäß Formel (1), in denen das Symbol Y für Stickstoff, Phosphor oder P=O steht, besonders bevorzugt für Stickstoff oder Phosphor, ganz besonders bevorzugt für Stickstoff.

Bevorzugt sind weiterhin Verbindungen gemäß Formel (1), in denen die Symbole Ar¹, Ar², Ar³, Ar⁴, Ar⁵ und Ar⁶ gleich oder verschieden bei jedem Auftreten für eine bivalente Aryl- oder Heteroarylgruppe mit 2 bis 16 C-Atomen stehen, die mit einem oder zwei Resten R¹ substituiert sein kann, besonders bevorzugt für eine bivalente Aryl- oder Heteroarylgruppe mit 4 bis 14 C-Atomen, die mit einem oder zwei Resten R¹ substituiert sein kann, ganz besonders bevorzugt für eine bivalente Aryl- oder Heteroarylgruppe, ausgewählt aus Benzol, Naphthalin, Anthracen, Phenanthren, Pyridin und Thiophen, insbesondere Benzol, die jeweils mit einem oder zwei Resten R¹ substituiert sein kann.

Bevorzugt sind weiterhin Verbindungen gemäß Formel (1), in denen die Symbole Ar⁷ Ar⁸ und Ar⁹ gleich oder verschieden bei jedem Auftreten für eine monovalente Aryl- oder Heteroarylgruppe mit 2 bis 16 C-Atomen stehen, die mit einem oder mehreren Resten R¹ substituiert sein kann, besonders bevorzugt für eine monovalente Aryl- oder Heteroarylgruppe mit 4 bis 14 C-Atomen, die mit einem oder mehreren Resten R¹ substituiert sein kann, ganz besonders bevorzugt für eine monovalente Aryl- oder Heteroarylgruppe, ausgewählt aus Benzol, Naphthalin, Anthracen, Phenanthren, Pyridin und Thiophen, insbesondere Benzol, die jeweils mit einem Rest R¹ substituiert sein kann, insbesondere mit einem aromatischen Rest R¹.

Bevorzugt sind weiterhin Verbindungen gemäß Formel (1), in denen das Symbol R gleich oder verschieden bei jedem Auftreten für H, CN, eine geradkettige oder verzweigte Alkylgruppe mit 1 bis 4 C-Atomen, wobei eine oder mehrere nicht benachbarte CH₂-Gruppen durch -R²C=CR²-, -C≡C-, -O- oder -S- ersetzt sein können und wobei ein oder mehrere H-Atome durch F ersetzt sein können, oder eine monovalente Aryl- oder Heteroarylgruppe mit 2 bis 16 C-Atomen, die mit einem oder mehreren Resten R¹ substituiert sein kann, steht, besonders bevorzugt für H, CN, Methyl oder eine monovalente Aryl- oder Heteroarylgruppe mit 4 bis 6 C-Atomen, die mit einem oder mehreren Resten R¹ substituiert sein kann, ganz besonders bevorzugt für H.

Bevorzugt sind weiterhin Verbindungen gemäß Formel (1), in denen das Symbol R¹ gleich oder verschieden bei jedem Auftreten für H, F, CN, eine geradkettige, verzweigte oder cyclische Alkyl- oder Alkoxygruppe mit 1 bis 10 C-Atomen, die mit einem oder mehreren Resten R² substituiert sein kann, wobei eine oder mehrere nicht benachbarte CH₂-Gruppen durch -R²C=CR²-, -C≡C-, Si(R²)₂, C=O, -O- oder -S- ersetzt sein können und wobei ein oder mehrere H-Atome durch F ersetzt sein können, oder eine Aryl- oder Heteroarylgruppe mit 2 bis 16 C-Atomen, die durch einen oder mehrere Reste R² substituiert sein kann, oder eine Aryloxy- oder Heteroaryloxygruppe mit 2 bis 16 aromatischen C-Atomen, die durch einen oder mehrere Reste R² substituiert sein kann, oder eine Kombination aus zwei, drei oder vier dieser Systeme steht; dabei können auch zwei oder mehrere Substituenten R¹ sowohl am selben Ring wie auch an unterschiedlichen Ringen miteinander ein mono- oder polycyclisches, aliphatisches oder aromatisches Ringsystem bilden.

Bevorzugt sind weiterhin Verbindungen, in denen der Index n gleich oder verschieden bei jedem Auftreten für 1, 2 oder 3 steht, bevorzugt für 1 oder 2, ganz besonders bevorzugt für 1.

Die bivalenten Aryl- bzw. Heteroarylgruppen Ar¹ und Ar² bzw. Ar³ und Ar⁴ bzw. Ar⁵ und Ar⁶ sind bevorzugt so verknüpft, dass eine gerade Anzahl aromatischer Ringatome zwischen den beiden Verknüpfungsstellen liegt, insbesondere eine durch vier teilbare Anzahl an Ringatomen. So sind beispielsweise für Phenylensysteme die ortho- und die para-Verknüpfung bevorzugt, insbesondere die para-Verknüpfung.

Bevorzugt sind Verbindungen gemäß Formel (1), in denen Ar¹, Ar³ und Ar⁵ für dieselbe Aryl- oder Heteroarylgruppe stehen. Weiterhin bevorzugt sind Verbindungen gemäß Formel (1), in denen Ar², Ar⁴ und Ar⁶ für dieselbe Aryl- oder Heteroarylgruppe stehen. Weiterhin bevorzugt sind Verbindungen gemäß Formel (1), in denen Ar⁷, Ar⁸ und Ar⁹ für dieselbe Aryl- oder Heteroarylgruppe stehen. Besonders bevorzugt sind Verbindungen gemäß Formel (1), die symmetrisch aufgebaut sind und die eine dreizählige Drehachse (C3) aufweisen, was sich nicht nur auf die aromatischen Gruppen Ar¹ bis Ar⁹ bezieht, sondern auch auf die Reste R, R¹ und R². Diese Bevorzugung ist durch die leichtere synthetische Zugänglichkeit der Verbindungen zu begründen. Jedoch auch die unsymmetrischen Verbindungen sind in mehr Stufen zugänglich.

Wenn die Verbindungen Atropisomerie um eine oder um mehrere Bindungen zeigen kann, so sind jeweils auch die isolierten oder angereicherten Atropisomere Gegenstand der Erfindung. Dies bezieht sich sowohl auf Enantiomere wie auch auf Diastereomere. Durch die Wahl geeigneter Atropisomere lässt sich beispielsweise die Löslichkeit der Verbindung beeinflussen.

Beispiele für bevorzugte Verbindungen gemäß Formel (1) sind die im Folgenden abgebildeten Beispiele 1 bis 42.

| | |
|---|---|
| 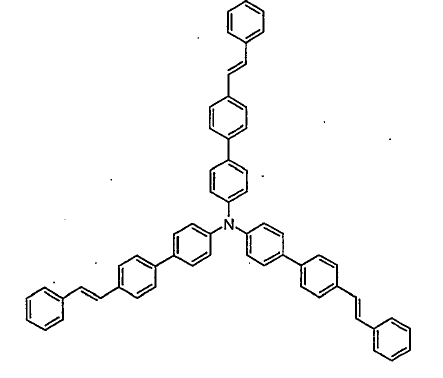 | 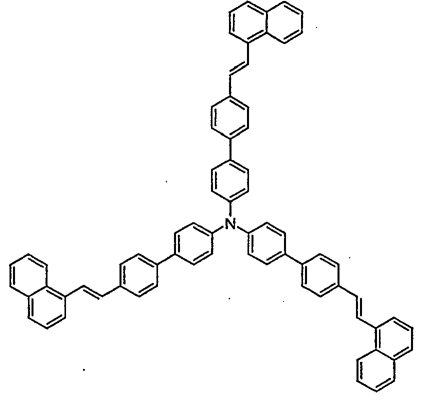 |
| Beispiel 1 | Beispiel 2 |
| 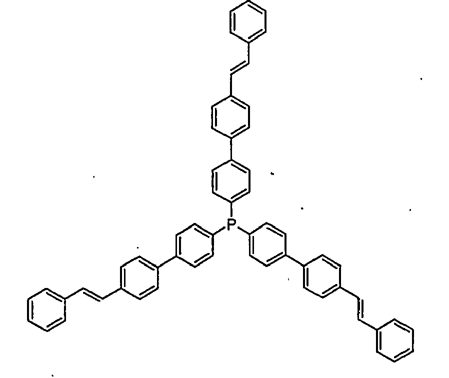 | 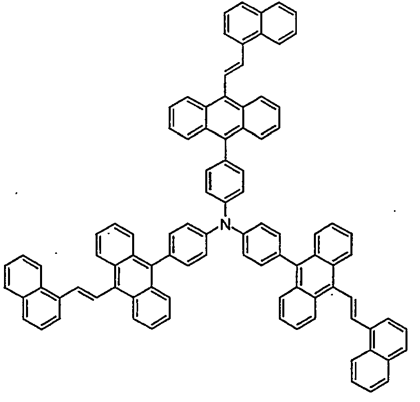 |
| Beispiel 3 | Beispiel 4 |
| 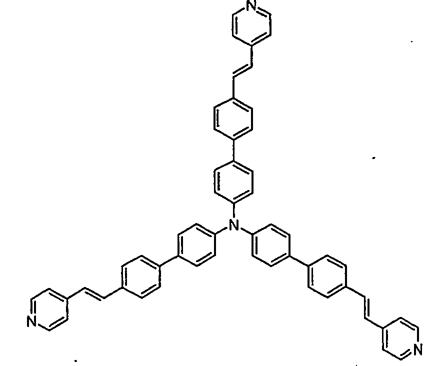 | 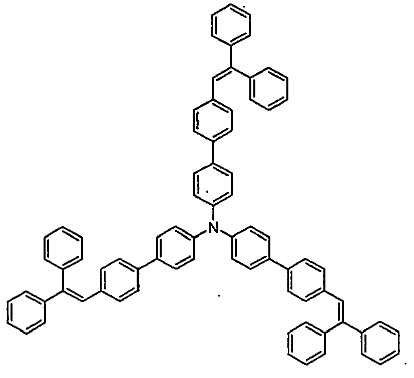 |
| Beispiel 5 | Beispiel 6 |
| 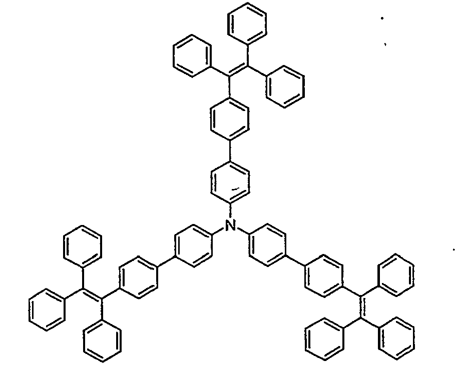 | 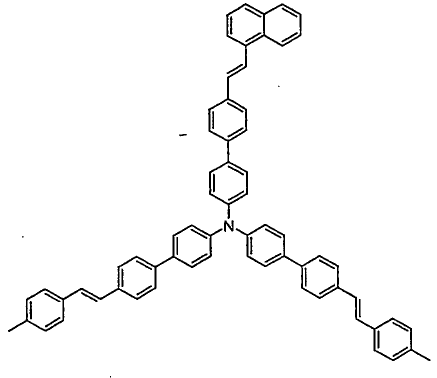 |
| Beispiel 7 | Beispiel 8 |
| 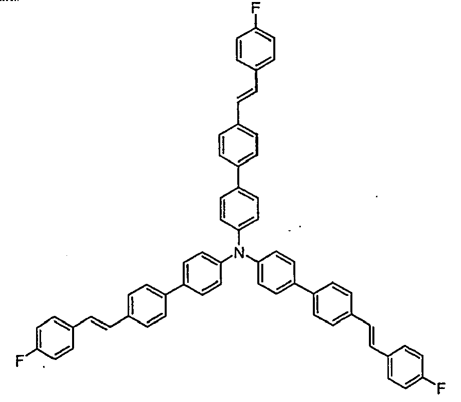 | 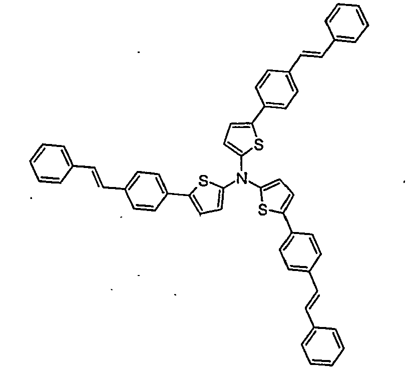 |
| Beispiel 9 | Beispiel 10 |
| 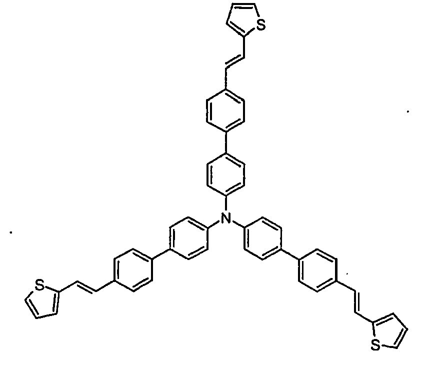 | 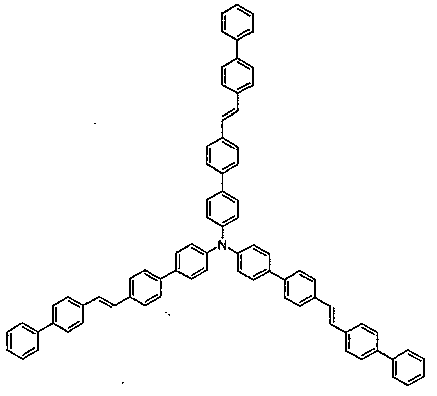 |
| Beispiel 11 | Beispiel 12 |
| 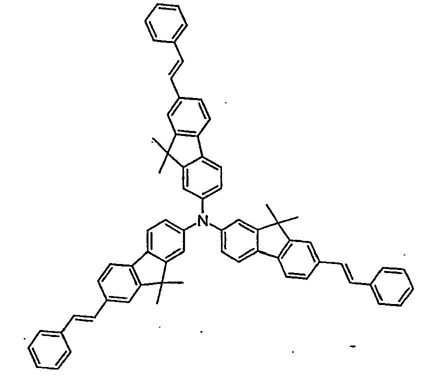 | 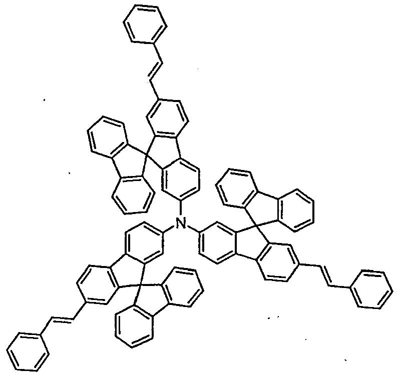 |
| Beispiel 13 | Beispiel 14 |
| 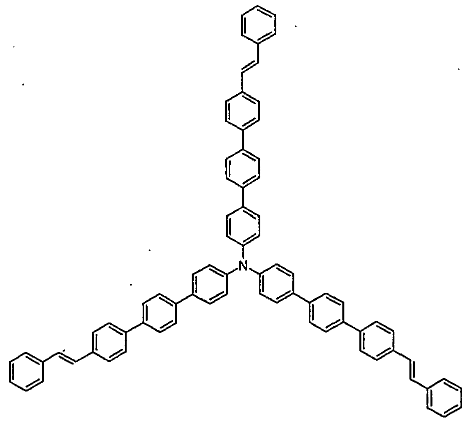 | 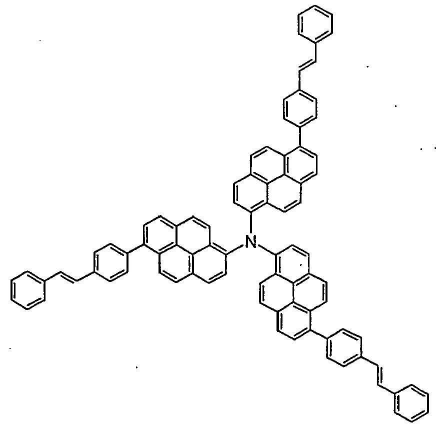 |
| Beispiel 15 | Beispiel 16 |
| 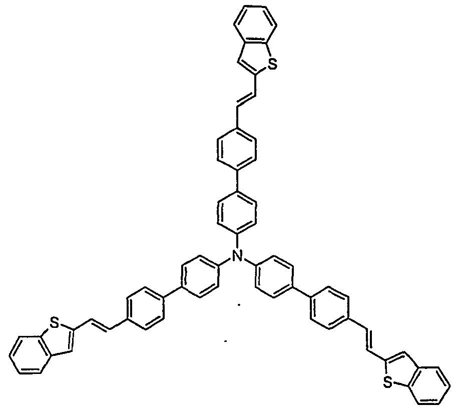 | 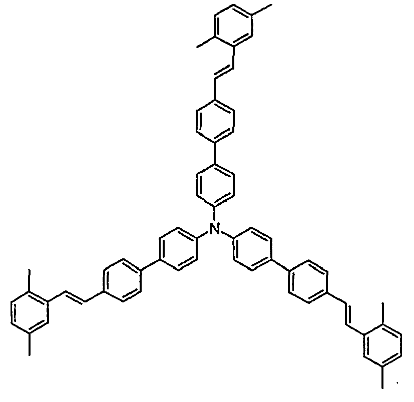 |
| Beispiel 17 | Beispiel 18 |
| 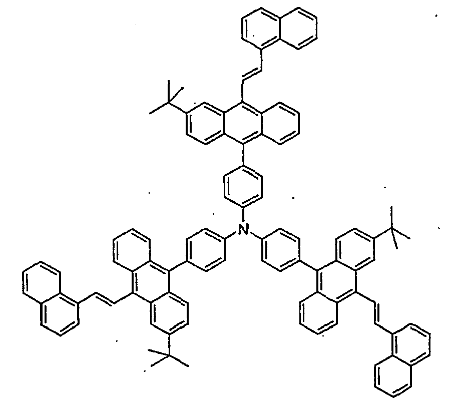 | 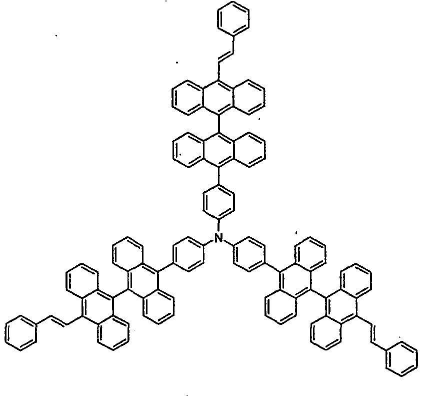 |
| Beispiel 19 | Beispiel 20 |
| 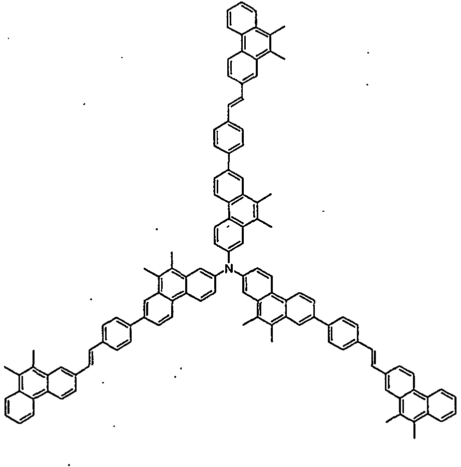 | 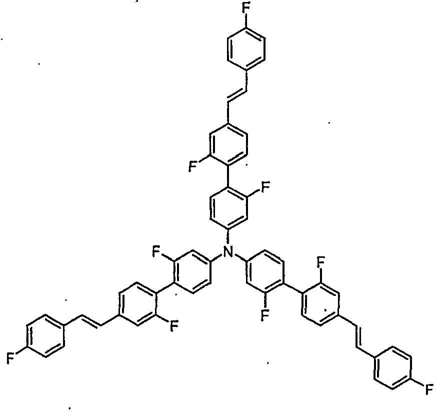 |
| Beispiel 21 | Beispiel 22 |
| 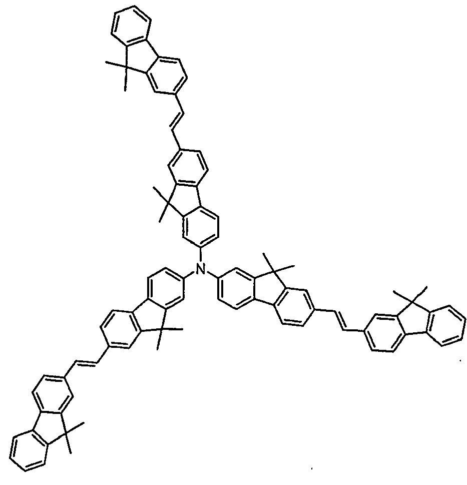 | 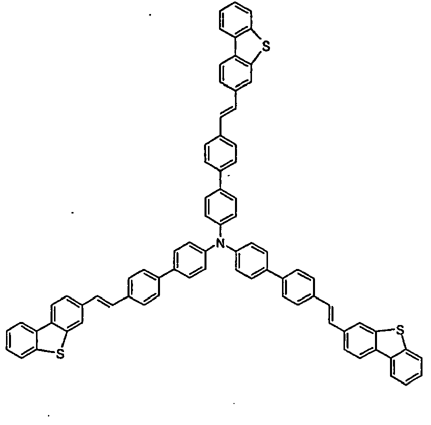 |
| Beispiel 23 | Beispiel 24 |
| 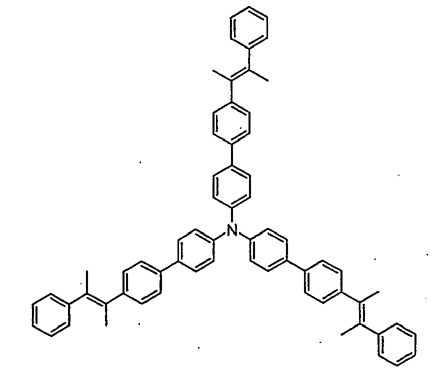 | 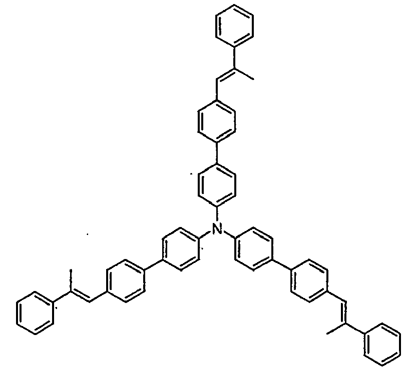 |
| Beispiel 25 | Beispiel 26 |
| 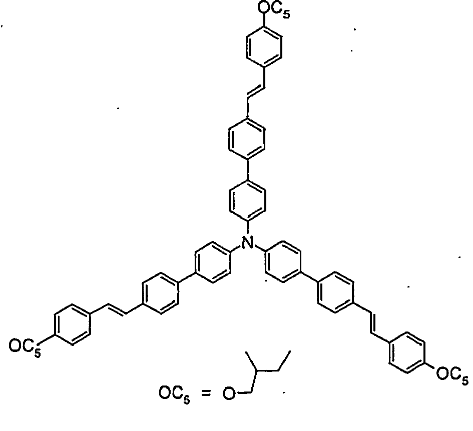 | 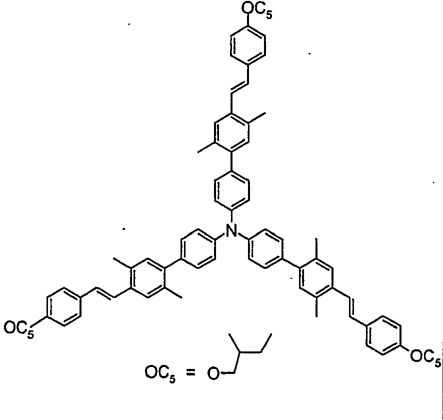 |
| Beispiel 27 | Beispiel 28 |
| 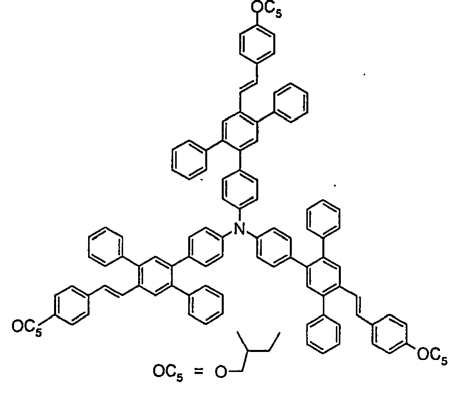 | 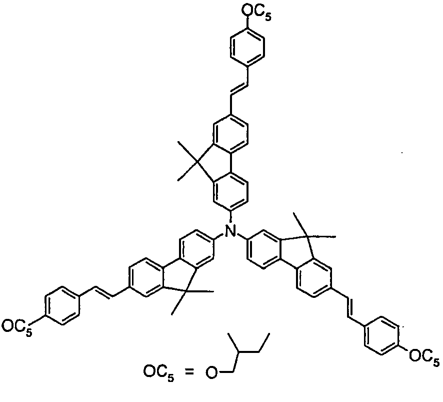 |
| Beispiel 29 | Beispiel 30 |
| 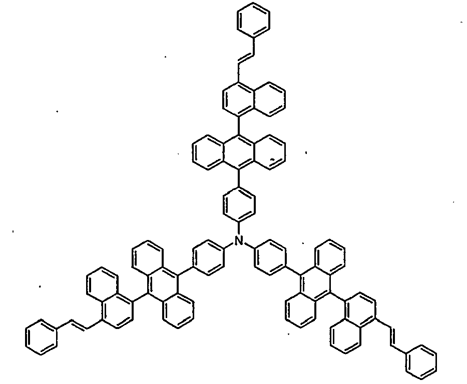 | 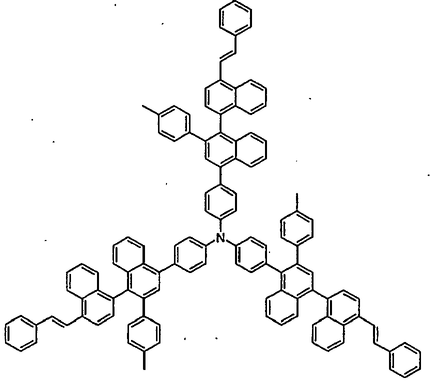 |
| Beispiel 31 | Beispiel 32 |
| 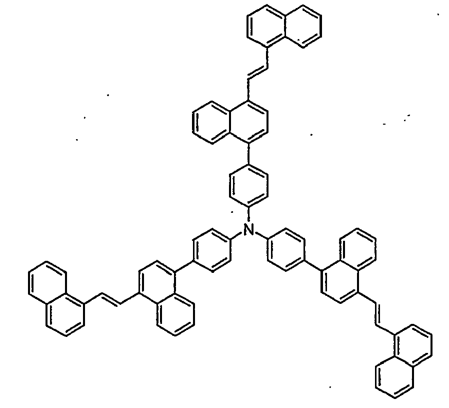 | 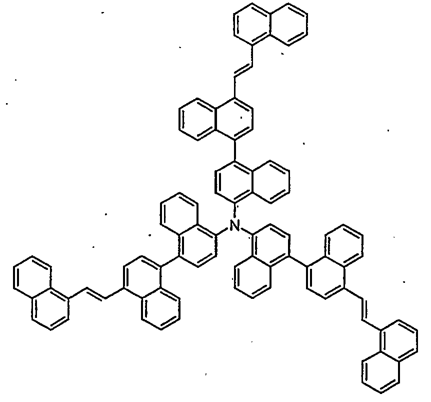 |
| Beispiel 33 | Beispiel 34 |
| 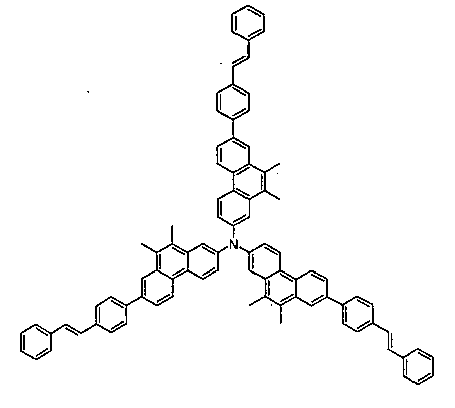 | 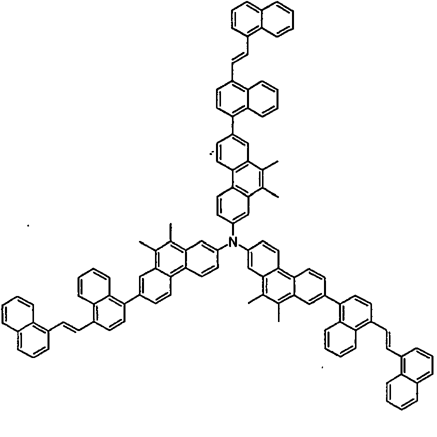 |
| Beispiel 35 | Beispiel 36 |
| 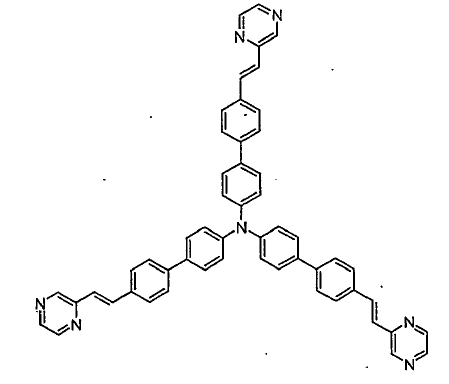 | 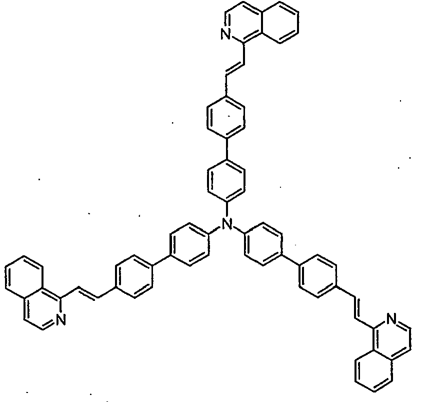 |
| Beispiel 37 | Beispiel 38 |
| 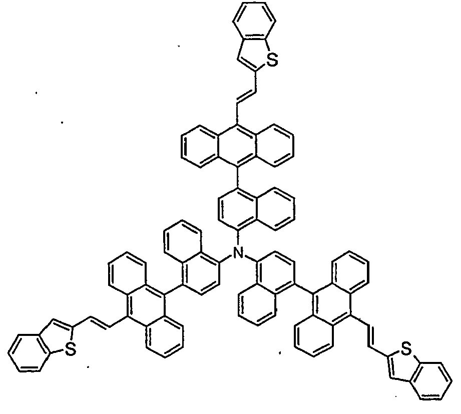 | 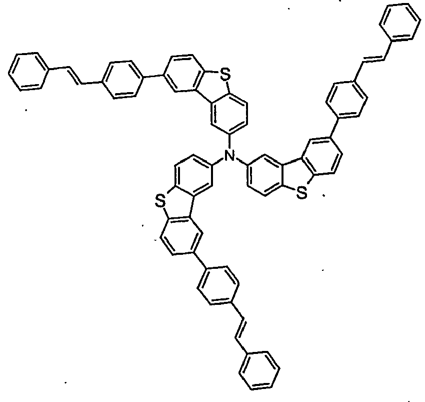 |
| Beispiel 39 | Beispiel 40 |
| 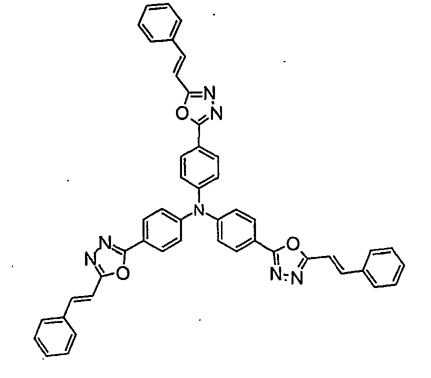 | 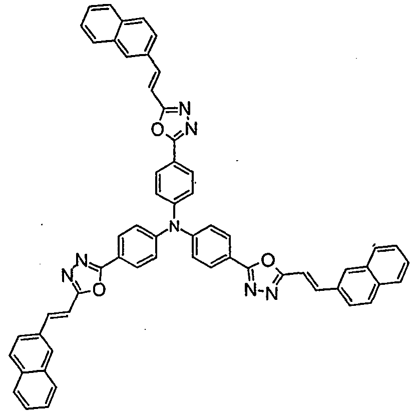 |
| Beispiel 41 | Beispiel 42 |

Die erfindungsgemäßen Verbindungen können nach dem Fachmann bekannten Syntheseschritten, wie z. B. Bromierung, Suzuki-Kupplung, Wittig-Horner-Reaktion, etc., dargestellt werden. So führt die Bromierung von leicht verfügbaren Triarylaminen zu Tris-p-brom-substituierten Triarylaminen, wobei hier - bedingt durch den starken +M-dirigierenden Effekt des Stickstoffatoms - häufig sehr gute Ausbeuten bei exzellenten Regioselektivitäten erreicht werden. Als Bromierungsagens können neben elementarem Brom vor allen auch N-BromVerbindungen wie N-Brom-succinimid (NBS) verwendet werden. Die so dargestellten Tris-p-brom-substituierten Triarylamine lassen leicht z. B. durch Suzuki-Kupplung unter Standardbedingungen mit funktionalisierten Arylboronsäuren in ausgezeichneten Ausbeuten umsetzen. Als Funktionalisierung kommen insbesondere Formyl-, Alkylcarbonyl- und Arylcarbonyl-Gruppen oder deren geschützte Analoga, z. B. in Form der entsprechenden Dioxolane, in Frage. Selbstverständlich können auch andere Kupplungsreaktionen (z. B. Stille-Kupplung, etc.) Verwendung finden. Die so erhaltenen Carbonyl-Substrate können dann leicht, z. B. durch eine Wittig-Horner-Reaktion, in die entsprechenden Olefine überführt werden.

Die Verbindungen gemäß Formel (1) können in organischen Elektrolumineszenzvorrichtungen eingesetzt werden. Dabei wird die Verbindung bevorzugt in der emittierenden Schicht als Mischung mit mindestens einem Hostmaterial eingesetzt. Es ist bevorzugt, wenn die Verbindung gemäß Formel (1) in der Mischung die emittierende Verbindung (der Dotand) ist. Bevorzugte Hostmaterialien sind organische Verbindungen, deren Emission kürzerwellig ist als die der Verbindung gemäß Formel (1) oder die überhaupt nicht im sichtbaren

Bereich emittieren. Auch die Verwendung der Verbindungen gemäß Formel (1) als Lochtransportmaterial ist möglich.

Als Hostmaterialien kommen verschiedene Stoffklassen in Frage. Bevorzugte Hostmaterialien sind ausgewählt aus den Klassen der Oligo-arylene (z. B. 2,2',7,7'-tetraphenyl-spirobifluoren gemäß EP 676461 oder Dinaphthylanthracen), insbesondere der Oligo-arylene enthaltend kondensierte aromatische Gruppen, der Oligo-arylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß der nicht offen gelegten Anmeldung DE 102004008304.5) oder der Atropisomere (z. B. gemäß der nicht offen gelegten Anmeldung EP 04026402.0). Besonders bevorzugte Hostmaterialien sind ausgewählt aus den Klassen der Oligoarylene, enthaltend Naphthalin, Anthracen und/oder Pyren oder Atropisomeren dieser Verbindungen, der Oligo-arylenvinylene, der Ketone, der Phosphinoxide und der Sulfoxide. Ganz besonders bevorzugte Hostmaterialien sind ausgewählt aus den Klassen der Oligoarylene, enthaltend Anthracen und/oder Pyren oder Atropisomeren dieser Verbindungen, der Phosphinoxide und der Sulfoxide.

Der Anteil der Verbindung gemäß Formel (1) in der Mischung beträgt zwischen 0.1 und 99.0 Gew.%, bevorzugt zwischen 0.5 und 50.0 Gew.%, besonders bevorzugt zwischen 1.0 und 20.0 Gew.%, insbesondere zwischen 1.0 und 10.0 Gew.%. Entsprechend beträgt der Anteil des Hostmaterials in der Mischung zwischen 1.0 und 99.9 Gew.%, bevorzugt zwischen 50.0 und 99.5 Gew.%, besonders bevorzugt zwischen 80.0 und 99.0 Gew.%, insbesondere zwischen 90.0 und 99.0 Gew.%.

Weiterhin bevorzugt sind organische Elektrolumineszenzvorrichtungen, **dadurch gekennzeichnet, dass** mehrere emittierende Verbindungen in derselben Schicht oder in unterschiedlichen Schichten verwendet werden, wobei mindestens eine dieser Verbindungen eine Struktur gemäß Formel (1) aufweist. Besonders bevorzugt weisen diese Verbindungen insgesamt mehrere Emissionsmaxima zwischen 380 nm und 750 nm auf, so dass insgesamt weiße Emission resultiert, d. h. außer der Verbindung gemäß Formel (1) wird noch mindestens eine weitere emittierende Verbindung verwendet, die fluoreszieren oder phosphoreszieren kann und die gelbes, orange oder rotes Licht emittiert. Insbesondere bevorzugt sind Dreischichtsysteme, wovon mindestens eine dieser Schichten eine Verbindung gemäß Formel (1) enthält und wobei die Schichten blaue, grüne und orange oder rote Emission zeigen (für den prinzipiellen Aufbau siehe z. B. WO 05/011013).

Außer Kathode, Anode und der emittierenden Schicht kann die organische Elektrolumineszenzvorrichtung noch weitere Schichten enthalten. Diese können beispielsweise sein: Lochinjektionsschicht, Lochtransportschicht, Elektronentransportschicht und/oder Elektroneninjektionsschicht. Es sei aber an dieser Stelle darauf hingewiesen, dass nicht notwendigerweise jede dieser Schichten vorhanden sein muss. So werden insbesondere bei Verwendung von Verbindungen gemäß Formel (1) mit elektronenleitenden Hostmaterialien weiterhin sehr gute Ergebnisse erhalten, wenn die organische Elektrolumineszenzvorrichtung keine separate Elektronentransportschicht enthält und die emittierende Schicht direkt an die Elektroneninjektionsschicht oder an die Kathode grenzt. Alternativ kann das Hostmaterial auch gleichzeitig in einer Elektronentransportschicht als Elektronentransportmaterial dienen. Ebenfalls kann es bevorzugt sein, wenn die organische Elektrolumineszenzvorrichtung keine separate Lochtransportschicht enthält und die emittierende Schicht direkt an die Lochinjektionsschicht oder an die Anode grenzt. Weiterhin kann es bevorzugt sein, wenn die Verbindung gemäß Formel (1) nicht oder nicht nur als Dotand in der emittierenden Schicht, sondern auch als lochleitende Verbindung (als Reinsubstanz oder als Mischung) in einer Lochtransportschicht verwendet wird.

Weiterhin bevorzugt ist eine organische Elektrolumineszenzvorrichtung, **dadurch gekennzeichnet, dass** eine oder mehrere Schichten mit einem Sublimationsverfahren beschichtet werden. Dabei werden die Materialien in Vakuum-Sublimationsanlagen bei einem Druck kleiner 10⁻⁵ mbar, bevorzugt kleiner 10⁻⁶ mbar, besonders bevorzugt kleiner 10⁻⁷ mbar aufgedampft.

Bevorzugt ist ebenfalls eine organische Elektrolumineszenzvorrichtung, **dadurch gekennzeichnet, dass** eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht.

Weiterhin bevorzugt ist eine organische Elektrolumineszenzvorrichtung, **dadurch gekennzeichnet, dass** eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Hohe Löslichkeit lässt sich entweder durch geeignete Substitution der Verbindungen oder aber auch durch die Wahl geeigneter Atropisomere erreichen.
Die erfindungsgemäßen Verbindungen weisen bei Verwendung in organischen Elektrolumineszenzvorrichtungen folgende überraschende Vorteile gegenüber dem Stand der Technik auf:
1. Die Effizienz entsprechender Vorrichtungen wird höher im Vergleich zu Systemen gemäß dem Stand der Technik, insbesondere im Vergleich zu Systemen, die zwischen der Gruppe Y und der Vinylgruppe nur eine statt erfindungsgemäß zwei oder mehr Aryl- oder Heteroarylgruppen enthalten.
2. Die Stabilität entsprechender Vorrichtungen wird höher im Vergleich zu Systemen gemäß dem Stand der Technik, was sich vor allem in einer deutlich höheren Lebensdauer zeigt.
3. Die Emissionsfarbe der Verbindungen ist tiefer blau im Vergleich zu den üblicherweise verwendeten Styrylaminen gemäß dem Stand der Technik. Dadurch eignen sie sich besser für die Verwendung in hochwertigen Vollfarbdisplays.
4. Die Verbindungen lassen sich gut und ohne erhebliche Zersetzung sublimieren und aufdampfen, sind dadurch leichter zu verarbeiten und deshalb besser für die Verwendung in OLEDs geeignet als Materialien gemäß dem Stand der Technik.

Im vorliegenden Anmeldetext und auch in den im Weiteren folgenden Beispielen wird auf die Verwendung erfindungsgemäßer Verbindungen in Bezug auf OLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch für weitere Verwendungen in anderen elektronischen Vorrichtungen zu benutzen, z. B. für organische Feld-Effekt-Transistoren (O-FETs), organische Dünnfilmtransistoren (O-TFTs), organische lichtemittierende Transistoren (O-LETs), organische integrierte Schaltungen (O-ICs), organische Solarzellen (O-SCs), organische Feld-Quench-Devices (O-FQDs) oder auch organische Laserdioden (O-Laser), um nur einige Anwendungen zu nennen. Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung durch die nachfolgenden Beispiele näher erläutert wird, ohne sie dadurch einschränken zu wollen.

### Beispiele:

Die nachfolgenden Synthesen wurden, sofern nicht anders angegeben, unter einer Schutzgasatmosphäre durchgeführt. Die Edukte wurden von den Firmen ALDRICH bzw. ABCR (Tris(4-bromphenyl)amin, 4-Formylbenzolboronsäure, Palladium(II)acetat, Tri-o-tolylphosphin, Anorganika, Lösemittel) bezogen.

### Beispiel 1: Synthese von Tris-1-[(1'-styryl)-4,4'-biphenyl]amin

### a) Synthese von Tris-1-(1'-formyl-4,4'-biphenyl)amin

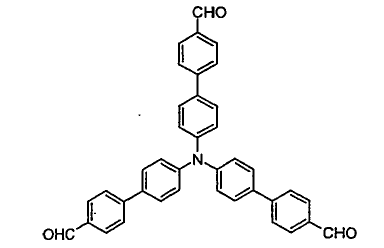

Ein entgaste Suspension von 48.2 g (100 mmol) Tris(4-bromphenyl)amin, 67.4 g (450 mmol) 4-Formylbenzolboronsäure und 156.4 g (630 mmol) Kaliumphosphathydrat in einem Gemisch aus 130 ml Dioxan, 300 ml Toluol und 375 ml Wasser wurde unter gutem Rühren mit 5.5 g (18 mmol) Tri-o-tolylphosphin und dann mit 674 mg (3 mmol) Palladium(II)acetat versetzt. Nach 5 h Kochen unter Rückfluss ließ man die Mischung erkalten. Der gelbgrüne Niederschlag wurde abgesaugt, dreimal mit 200 ml Ethanol / Wasser (1:1, v:v) und dreimal mit 100 ml Ethanol gewaschen und anschließend im Vakuum getrocknet. Ausbeute: 51.9 g (93 mmol), 93.0 % d. Th., Reinheit nach ¹H-NMR ca. 98%.
¹H-NMR (CDCl₃): δ [ppm] = 10.05 (s, 3H, CHO), 7.95 und 7.76 (2 x d, ³J_{HH} = 8.3 Hz, 12H), 7.61 und 7.28 (2 x d, ³J_{HH} = 8.3 Hz, 12H).

### b) Synthese von Tris-1-[(1'-styryl)-4,4'-biphenyl]amin

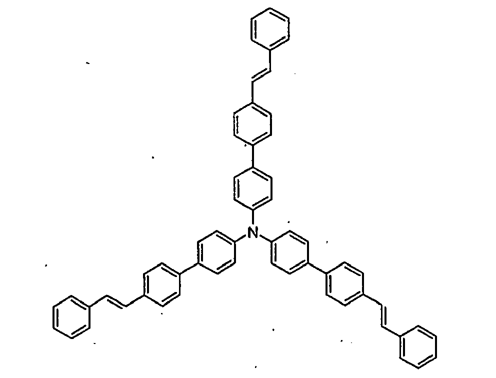

Ein Gemisch aus 50.0 ml (240 mmol) Phenylmethanphosphonsäurediethylester und 1000 ml DMF wurde unter gutem Rühren bei 0 °C mit 46.1 g (480 mmol) Natrium*tert*-butylat versetzt. Zu dieser Mischung wurde bei 0-10 °C eine 30 °C warme Lösung von 37.0 g (66 mmol) Tris-1-(1'-formyl-4,4'-biphenyl)amin in 1500 ml DMF langsam zugetropft. Nach vollendeter Zugabe wurde weitere 3 h bei 0 °C bis 10°C und 12 h bei Raumtemperatur gerührt. Anschließend wurden 100 ml 2.5 N HCl, dann 300 ml Wasser und dann 300 ml Ethanol zugegeben. Der gelbe feinkristalline Niederschlag wurde abgesaugt (P3), dreimal mit je 200 ml eines Gemischs aus Ethanol / Wasser (1:1, v:v) und dreimal mit je 200 ml Ethanol gewaschen. Der Feststoff wurde nach Trocknen im Vakuum sechsmal unter Lichtausschluss aus DMF (ca. 20 ml/g) umkristallisiert. Ausbeute: 27.2 g (35 mmol), 52.8 %, Reinheit nach HPLC > 99.9 %.
¹H-NMR (Tetrachlorethan-d2): δ [ppm] = 7.64-7.53 (m, 24H), 7.39-7.36 (m, 6H), 7.29-7.24 (m, 9H), 7.15 (br. s, 6H).

### Beispiel 2: Herstellung der OLEDs

Die Herstellung von OLEDs erfolgte nach einem allgemeinen Verfahren gemäß WO 04/058911, das im Einzelfall auf die jeweiligen Gegebenheiten (z. B. Schichtdickenvariation, um optimale Effizienz bzw. Farbe zu erreichen) angepasst wurde.

In den folgenden Beispielen werden die Ergebnisse verschiedener OLEDs vorgestellt. Der grundlegende Aufbau, die verwendeten Materialien und Schichtdicken, außer der emittierenden Schicht und der Lochtransportschicht, waren zur besseren Vergleichbarkeit identisch. Analog dem o. g. allgemeinen Verfahren wurden OLEDs mit folgendem Aufbau erzeugt:
- Lochinjektionsschicht (HIL): 20-600 nm PEDOT/ PSS (aus wässriger Dispersion aufgeschleudert; bezogen von H. C. Starck, Goslar, Deutschland; Poly(3,4-ethylendioxy-2,5-thiophen)+Polystyrolsulfonsäure)
- Lochtransportschicht (HTM): genauer Aufbau: siehe Tabelle 1 NaphDATA (aufgedampft; bezogen von SynTec, Wolfen, Deutschland; 4,4',4"-Tris(N-1-naphthyl-N-phenyl-amino)-triphenylamin) und/oder S-TAD (aufgedampft; hergestellt nach WO 99/12888; 2,2',7,7'-Tetrakis(diphenylamino)-spiro-9,9'-bifluoren) und/oder
20 nm NPB (aufgedampft; N-Naphthyl-N-phenyl-4,4'-diaminobiphenyl) und/oder
20 nm **HTM1** (aufgedampft; hergestellt nach WO 99/12888; 2,2',7,7'-Tetrakis(di-*para-*tolylamino)spiro-9,9'-bifluoren)
- Emissionschicht (EML): siehe Tabelle 1 für Materialien, Konzentration und Schichtdicken
- Elektronenleiter (ETL): 20 nm Alq₃ (bezogen von SynTec; Tris(chinolinato)aluminium(III))
- LiF-Al (Kathode): 1 nm LiF, darauf 150 nm Al

Diese noch nicht optimierten OLEDs wurden standardmäßig charakterisiert; hierfür wurden die Elektrolumineszenzspektren, die Effizienz (gemessen in cd/A), die Leistungseffizienz (gemessen in Im/W) in Abhängigkeit der Helligkeit, berechnet aus Strom-Spannungs-Leuchtdichte-Kennlinien (IUL-Kennlinien), und die Lebensdauer bestimmt. Als Lebensdauer wird die Zeit definiert, nach der die Anfangshelligkeit von 500cd/m²auf die Hälfte gesunken ist.

In Tabelle 1 sind die Ergebnisse einiger OLEDs (Beispiele 3 bis 6) zusammengefasst, wobei jeweils die Zusammensetzung der EML und der HTL inklusive der Schichtdicken mit aufgeführt ist. Beispiel 3 zeigt Vergleichsbeispiele, die in der Emissionsschicht entweder nur verschiedene üblicherweise verwendete Hostmaterialien enthalten oder den Dotanden **D2** gemäß dem oben genannten Stand der Technik in einem Hostmaterial. In den erfindungsgemäßen Beispielen 4 bis 6 enthalten die EMLs als emittierende Materialien gemäß Formel (1) den Dotanden **D1** (gemäß Beispiel 1). Als Hostmaterialien dienen die im Folgenden abgebildeten Verbindungen **H1** bis **H3**. Als Lochtransportmaterialien dienen jeweils zwei der oben genannten Materialien NaphDATA, S-TAD, NPB und **HTM1,** die in zwei Schichten übereinander aufgebracht werden. Zur besseren Übersichtlichkeit sind die entsprechenden Strukturformeln der verwendeten Dotanden und Hostmaterialien im Folgenden dargestellt:

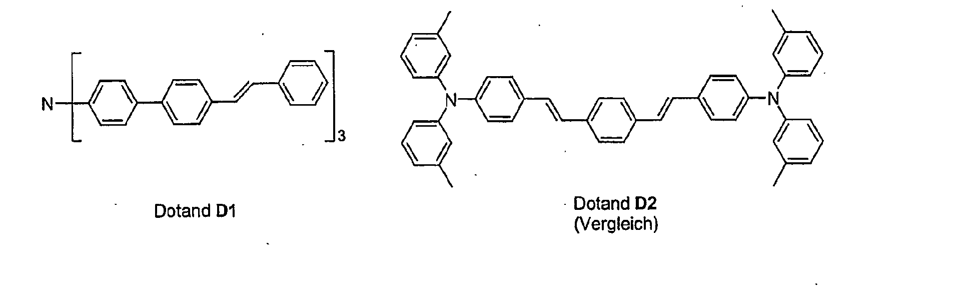

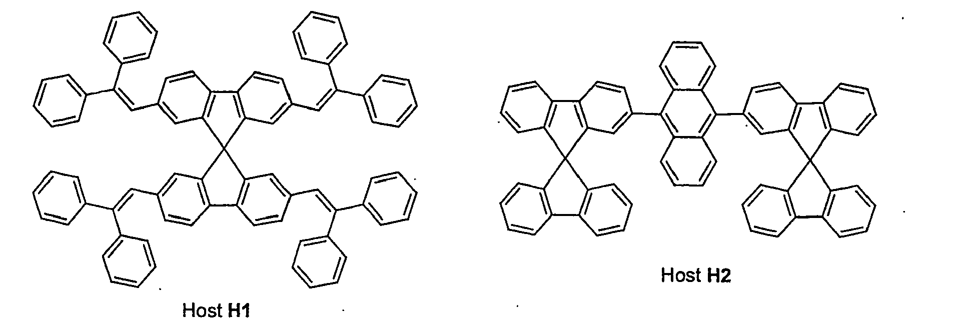

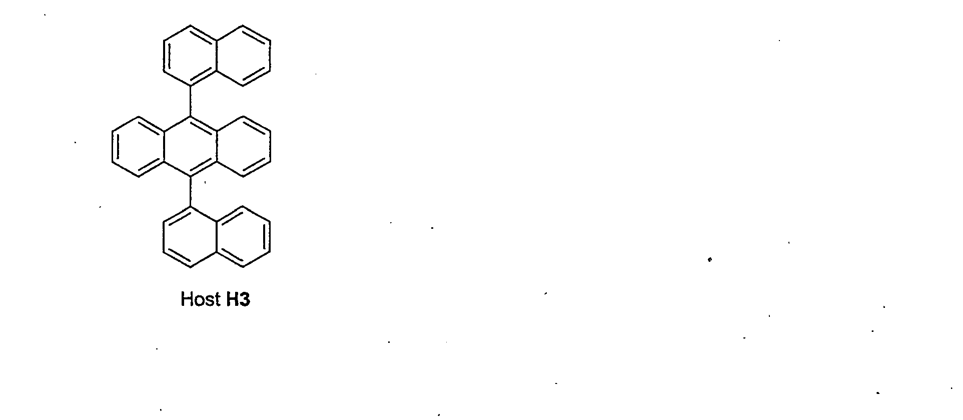

**Tabelle 1**

| Beispiel | HTL1 | HTL2 | EML | Max. Effizienz (cd/A) | Spannung (V) bei 100 cd/m² | CIE^{a} | Lebensdauer (h) ^{b} |
|---|---|---|---|---|---|---|---|
| **Beispiel 3a** | **NaphDATA** | **S-TAD** | **H1** | 4.2 | 5.8 | x=0.17 | 1400 |
| **(Vergleich)** | (20 nm) | (20 nm) | (30 nm) | | | y=0.26 | |
| **Beispiel 3b** | **NaphDATA** | **S-TAD** | **H1 : D2** (5 %) | 4.9 | 6.3 | x=0.17 | 1100 |
| **(Vergleich)** | (20 nm) | (20 nm) | (30 nm) | | | y=0.31 | |
| **Beispiel3c** | **NaphDATA** | **S-TAD** | **H2** | 3.3 | 6.5 | x=0.15 | 450 |
| **(Vergleich)** | (20 nm) | (20 nm) | (30 nm) | | | y=0.15 | |
| **Beispiel 3d** | **NaphDATA** | **S-TAD** | **H3** | 1.1 | 5.8 | x=0.17 | 800 |
| **(Vergleich)** | (20 nm) | (20 nm) | (30 nm) | | | y=0.19 | |
| **Beispiel 4a** | **NaphDATA** | **S-TAD** | **H3 : D1** (5 %) | 3.7 | 5.2 | x=0.15 | 1300 |
| | (20 nm) | (20 nm) | (30 nm) | | | y=0.10 | |
| **Beispiel 4b** | **NaphDATA** | **S-TAD** | **H3 : D1** (10 %) | 3.6 | 4.7 | x=0.15 | 1600 |
| | (20 nm) | (20 nm) | (30 nm) | | | y=0.13 | |
| **Beispiel 5** | **NaphDATA** | **NPB** | **H3 : D1** (2 %) | 5.2 | 5.3 | x=0.16 | 900 |
| | (20 nm) | (20 nm) | (30 nm) | | | y=0.11 | |
| **Beispiel 6a** | **HTM1** | **NPB** | **H3 : D1** (2 %) | 2.7 | 5.3 | x=0.16 | 3000 |
| | (20 nm) | (20 nm) | (30 nm) | | | y=0.10 | |
| **Beispiel 6b** | **HTM1** | **NPB** | **H3** : **D1** (5 %) | 3.2 | 5.1 | x=0.16 | 4100 |
| | (20 nm) | (20 nm) | (30 nm) | | | y=0.13 | |

| | | | | | | | |
|---|---|---|---|---|---|---|---|
| ^{a} CIE-Koordinaten: Farbkoordinaten der Commision Internationale de l'Eclairage 1931. ^{b} Lebensdauer: Zeit bis zum Abfall der Helligkeit auf 50 % der Anfangshelligkeit, gemessen bei einer Anfangshelligkeit von 500 cd/m². | | | | | | | |

Zusammenfassend kann gesagt werden, dass OLEDs, enthaltend emittierende Verbindungen gemäß Formel (1), eine längere Lebensdauer bei deutlich tiefer blauer Farbe aufweisen als Materialien gemäß dem Stand der Technik, wie man leicht Tabelle 1 entnehmen kann. Daher eignen sich diese Verbindungen besser für die Verwendung in OLEDs als Materialien gemäß dem Stand der Technik.

## Patentansprüche

1. Verbindungen gemäß Formel (1), 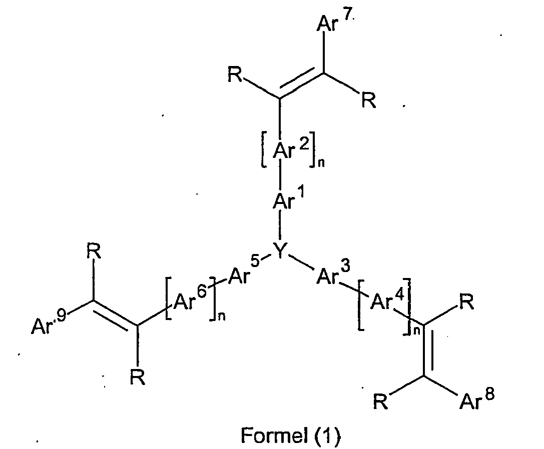 wobei für die verwendeten Symbole und Indizes gilt:
Y ist bei jedem Auftreten Stickstoff, Phosphor, Arsen, Antimon, P=O, As=O oder Sb=O;
Ar¹, Ar², Ar³, Ar⁴, Ar⁵, Ar⁶ ist bei jedem Auftreten gleich oder verschieden eine bivalente Aryl- oder Heteroarylgruppe mit 2 bis 24 C-Atomen, die mit einem oder mehreren Resten R¹ substituiert sein kann;
Ar⁷, Ar⁸, Ar⁹ ist bei jedem Auftreten gleich oder verschieden eine monovalente Aryl- oder Heteroarylgruppe mit 2 bis 24 C-Atomen, die mit einem oder mehreren Resten R¹ substituiert sein kann;
R ist bei jedem Auftreten gleich oder verschieden H, CN, eine geradkettige, verzweigte oder cyclische Alkylgruppe mit 1 bis 40 C-Atomen, die mit einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können, oder eine monovalente Aryl- oder Heteroarylgruppe mit 2 bis 24 C-Atomen, die mit einem oder mehreren Resten R¹ substituiert sein kann;
R¹ ist bei jedem Auftreten gleich oder verschieden H, F, Cl, Br, I, CN, NO₂, eine geradkettige, verzweigte oder cyclische Alkyl-, Alkoxy- oder Thioalkoxygruppe mit 1 bis 40 C-Atomen, die mit einem oder mehreren Resten R² substituiert sein kann, wobei eine oder mehrere nicht benachbarte CH₂-Gruppen durch -R²C=CR²-, -C≡C-, Si(R²)₂, Ge(R²)₂, Sn(R²)₂, C=O, C=S, C=Se, C=NR², -O-, -S- oder -CONR²- ersetzt sein können und wobei ein oder mehrere H-Atome durch F, Cl, Br, I, CN oder NO₂ ersetzt sein können, oder eine Aryl- oder Heteroarylgruppe mit 2 bis 24 C-Atomen, welche durch einen oder mehrere Reste R² substituiert sein kann, oder eine Aryloxy- oder Heteroaryloxygruppe mit 2 bis 24 aromatischen C-Atomen, die durch einen oder mehrere Reste R² substituiert sein kann, oder eine Kombination aus zwei, drei, vier oder fünf dieser Systeme; dabei können auch zwei oder mehrere Substituenten R¹ sowohl am selben Ring wie auch an unterschiedlichen Ringen miteinander ein mono- oder polycyclisches, aliphatisches oder aromatisches Ringsystem bilden;
R² ist bei jedem Auftreten gleich oder verschieden H oder ein aliphatischer oder aromatischer Kohlenwasserstoffrest mit 1 bis 20 C-Atomen;
n ist bei jedem Auftreten gleich oder verschieden 1, 2, 3, 4 oder 5.

2. Verbindungen gemäß Anspruch 1, **dadurch gekennzeichnet, dass** das Symbol Y für Stickstoff, Phosphor oder P=O steht.

3. Verbindungen gemäß Anspruch 1 und/oder 2, **dadurch gekennzeichnet, dass** die Symbole Ar¹, Ar², Ar³, Ar⁴, Ar⁵ und Ar⁶ gleich oder verschieden bei jedem Auftreten für eine bivalente Aryl- oder Heteroarylgruppe mit 2 bis 16 C-Atomen stehen, die mit einem oder zwei Resten R¹ substituiert sein kann.

4. Verbindungen gemäß einem oder mehreren der Ansprüche 1 bis 3, **dadurch gekennzeichnet, dass** die Symbole Ar⁷ Ar⁸ und Ar⁹ gleich oder verschieden bei jedem Auftreten für eine monovalente Aryl- oder Heteroarylgruppe mit 2 bis 16 C-Atomen stehen, die mit einem oder mehreren Resten R¹ substituiert sein kann.

5. Verbindungen gemäß einem oder mehreren der Ansprüche 1 bis 4, **dadurch gekennzeichnet, dass** das Symbol R gleich oder verschieden bei jedem Auftreten für H, CN, eine geradkettige oder verzweigte Alkylgruppe mit 1 bis 4 C-Atomen, wobei eine oder mehrere nicht benachbarte CH₂-Gruppen durch -R²C=CR²-, -C=C-, -O- oder -S- ersetzt sein können und wobei ein oder mehrere H-Atome durch F ersetzt sein können, oder eine monovalente Aryl- oder Heteroarylgruppe mit 2 bis 16 C-Atomen, die mit einem oder mehreren Resten R¹ substituiert sein kann, steht.

6. Verbindungen gemäß einem oder mehreren der Ansprüche 1 bis 5, **dadurch gekennzeichnet, dass** das Symbol R¹ gleich oder verschieden bei jedem Auftreten für H, F, CN, eine geradkettige, verzweigte oder cyclische Alkyl- oder Alkoxygruppe mit 1 bis 10 C-Atomen, die mit einem oder mehreren Resten R² substituiert sein kann, wobei eine oder mehrere nicht benachbarte CH₂-Gruppen durch -R²C=CR²-, -C≡C-, Si(R²)₂, C=O, -O- oder -S- ersetzt sein können und wobei ein oder mehrere H-Atome durch F ersetzt sein können, oder eine Aryl- oder Heteroarylgruppe mit 2 bis 16 C-Atomen, die durch einen oder mehrere Reste R² substituiert sein kann, oder eine Aryloxy- oder Heteroaryloxygruppe mit 2 bis 16 aromatischen C-Atomen, die durch einen oder mehrere Reste R² substituiert sein kann, oder eine Kombination aus zwei, drei oder vier dieser Systeme; dabei können auch zwei oder mehrere Substituenten R¹ sowohl am selben Ring wie auch an unterschiedlichen Ringen miteinander ein mono- oder polycyclisches, aliphatisches oder aromatisches Ringsystem bilden, steht.

7. Verbindungen gemäß einem oder mehreren der Ansprüche 1 bis 6, **dadurch gekennzeichnet, dass** der Index n gleich oder verschieden bei jedem Auftreten für 1, 2 oder 3 steht.

8. Verbindungen gemäß einem oder mehreren der Ansprüche 1 bis 7, **dadurch gekennzeichnet, dass** Ar¹, Ar³ und Ar⁵ für dieselbe Aryl- oder Heteroarylgruppe stehen.

9. Verbindungen gemäß einem oder mehreren der Ansprüche 1 bis 8, **dadurch gekennzeichnet, dass** Ar², Ar⁴ und Ar⁶ für dieselbe Aryl- oder Heteroarylgruppe stehen.

10. Verbindungen gemäß einem oder mehreren der Ansprüche 1 bis 9, **dadurch gekennzeichnet, dass** Ar⁷, Ar⁸ und Ar⁹ für dieselbe Aryl- oder Heteroarylgruppe stehen.

11. Verbindungen gemäß einem oder mehreren der Ansprüche 1 bis 10, **dadurch gekennzeichnet, dass** sie symmetrisch aufgebaut sind und eine dreizählige Drehachse (C3) aufweisen.

12. Verwendung von Verbindungen gemäß einem oder mehreren der Ansprüche 1 bis 11 in organischen elektronischen Vorrichtungen.

13. Organische elektronische Vorrichtungen, enthaltend mindestens eine Verbindung gemäß einem oder mehreren der Ansprüche 1 bis 11, ausgewählt aus der Gruppe der organischen Elektrolumineszenzvorrichtungen (OLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs) oder organischen Laserdioden (O-Laser).

14. Organische Elektrolumineszenzvorrichtung, enthaltend Anode, Kathode und mindestens eine emittierende Schicht, **dadurch gekennzeichnet, dass** die emittierende Schicht mindestens eine Verbindung gemäß einem oder mehreren der Ansprüche 1 bis 11 enthält.

15. Organische Elektrolumineszenzvorrichtung gemäß Anspruch 14, **dadurch gekennzeichnet, dass** die Verbindung gemäß einem oder mehreren der Ansprüche 1 bis 11 als Mischung mit mindestens einem Hostmaterial eingesetzt wird.

16. Organische Elektrolumineszenzvorrichtung gemäß Anspruch 15, **dadurch gekennzeichnet, dass** das Hostmaterial ausgewählt ist aus den Klassen der Oligo-arylene, der Oligo-arylene enthaltend kondensierte aromatische Gruppen, der Oligo-arylenvinylene, der polypodalen Metallkomplexe, der lochleitenden Verbindungen, der elektronenleitenden Verbindungen, der Ketone, der Phosphinoxide, der Sulfoxide oder der Atropisomere.

17. Organische Elektrolumineszenzvorrichtung gemäß Anspruch 15 und/oder 16, **dadurch gekennzeichnet, dass** der Anteil der Verbindung gemäß einem oder mehreren der Ansprüche 1 bis 11 in der Mischung zwischen 0.1 und 99.0 Gew.% beträgt und der Anteil des Hostmaterials entsprechend zwischen 1.0 und 99.9 Gew.% beträgt.

18. Organische Elektrolumineszenzvorrichtung gemäß einem oder mehreren der Ansprüche 14 bis 17, **dadurch gekennzeichnet, dass** noch weitere Schichten vorhanden sind, ausgewählt.aus Lochinjektionsschicht, Lochtransportschicht, Elektronentransportschicht und/oder Elektroneninjektionsschicht.

19. Organische Elektrolumineszenzvorrichtung gemäß einem oder mehreren der Ansprüche 14 bis 18, **dadurch gekennzeichnet, dass** mehrere emittierende Verbindungen in derselben Schicht oder in unterschiedlichen Schichten verwendet werden, wovon mindestens eine dieser Verbindungen eine Struktur gemäß Formel (1) aufweist.

## Claims

1. Compounds of the formula (1) 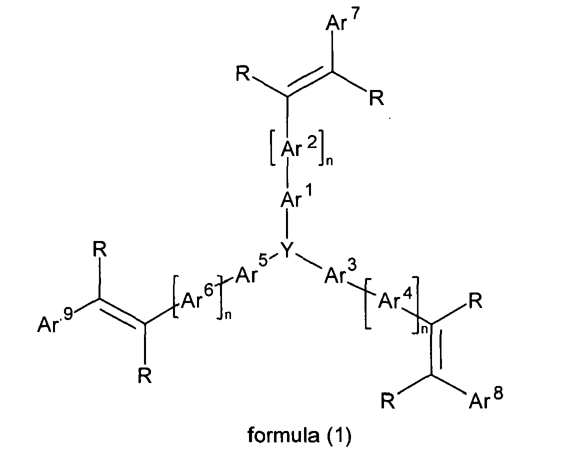 where the following applies to the symbols and indices used:
Y is on each occurrence nitrogen, phosphorus, arsenic, antimony, P=O, As=O or Sb=O;
Ar¹, Ar², Ar³, Ar⁴, Ar⁵, Ar⁶ are on each occurrence, identically or differently, a divalent aryl or heteroaryl group having 2 to 24 C atoms, which may be substituted by one or more radicals R¹;
Ar⁷, Ar⁸, Ar⁹ are on each occurrence, identically or differently, a monovalent aryl or heteroaryl group having 2 to 24 C atoms, which may be substituted by one or more radicals R¹;
R is on each occurrence, identically or differently, H, CN, a straight-chain, branched or cyclic alkyl group having 1 to 40 C atoms, which may be substituted by one or more radicals R², where one or more non-adjacent CH₂ groups may be replaced by -R²C=CR²-, -C≡C-, Si(R²)₂, Ge(R²)₂, Sn(R²)₂, C=O, C=S, C=Se, C=NR², -O-, -S- or -CONR²- and where one or more H atoms may be replaced by F, Cl, Br, I, CN or NO₂, or a monovalent aryl or heteroaryl group having 2 to 24 C atoms, which may be substituted by one or more radicals R¹;
R¹ is on each occurrence, identically or differently, H, F, Cl, Br, I, CN, NO₂, a straight-chain, branched or cyclic alkyl, alkoxy or thioalkoxy group having 1 to 40 C atoms, which may be substituted by one or more radicals R², where one or more non-adjacent CH₂ groups may be replaced by -R²C=CR²-, -C≡C-, Si(R²)₂, Ge(R²)₂, Sn(R²)₂, C=O, C=S, C=Se, C=NR², -O-, -S- or -CONR²- and where one or more H atoms may be replaced by F, Cl, Br, I, CN or NO₂, or an aryl or heteroaryl group having 2 to 24 C atoms, which may be substituted by one or more radicals R², or an aryloxy or heteroaryloxy group having 2 to 24 aromatic C atoms, which may be substituted by one or more radicals R², or a combination of two, three, four or five of these systems; two or more substituents R¹ here, both on the same ring and also on different rings, may also form a mono- or polycyclic, aliphatic or aromatic ring system with one another;
R² is on each occurrence, identically or differently, H or an aliphatic or aromatic hydrocarbon radical having 1 to 20 C atoms;
n is on each occurrence, identically or differently, 1, 2, 3, 4 or 5.

2. Compounds according to Claim 1, **characterised in that** the symbol Y stands for nitrogen, phosphorus or P=O.

3. Compounds according to Claim 1 and/or 2, **characterised in that** the symbols Ar¹, Ar², Ar³, Ar⁴, Ar⁵ and Ar⁶, identically or differently on each occurrence, stand for a divalent aryl or heteroaryl group having 2 to 16 C atoms, which may be substituted by one or two radicals R¹.

4. Compounds according to one or more of Claims 1 to 3, **characterised in that** the symbols Ar⁷, Ar⁸ and Ar⁹, identically or differently on each occurrence, stand for a monovalent aryl or heteroaryl group having 2 to 16 C atoms, which may be substituted by one or more radicals R¹.

5. Compounds according to one or more of Claims 1 to 4, **characterised in that** the symbol R, identically or differently on each occurrence, stands for H, CN, a straight-chain or branched alkyl group having 1 to 4 C atoms, where one or more non-adjacent CH₂ groups may be replaced by -R²C=CR²-, -C≡C-, -O- or -S- and where one or more H atoms may be replaced by F, or a monovalent aryl or heteroaryl group having 2 to 16 C atoms, which may be substituted by one or more radicals R¹.

6. Compounds according to one or more of Claims 1 to 5, **characterised in that** the symbol R¹, identically or differently on each occurrence, stands for H, F, CN, a straight-chain, branched or cyclic alkyl or alkoxy group having 1 to 10 C atoms, which may be substituted by one or more radicals R², where one or more non-adjacent CH₂ groups may be replaced by -R²C=CR²-, -C≡C-, Si(R²)₂, C=O, -O- or -S- and where one or more H atoms may be replaced by F, or an aryl or heteroaryl group having 2 to 16 C atoms, which may be substituted by one or more radicals R², or an aryloxy or heteroaryloxy group having 2 to 16 aromatic C atoms, which may be substituted by one or more radicals R², or a combination of two, three or four of these systems; two or more substituents R¹ here, both on the same ring and also on different rings, may also form a mono- or polycyclic, aliphatic or aromatic ring system with one another.

7. Compounds according to one or more of Claims 1 to 6, **characterised in that** the index n, identically or differently on each occurrence, stands for 1, 2 or 3.

8. Compounds according to one or more of Claims 1 to 7, **characterised in that** Ar¹, Ar³ and Ar⁵ stand for the same aryl or heteroaryl group.

9. Compounds according to one or more of Claims 1 to 8, **characterised in that** Ar², Ar⁴ and Ar⁶ stand for the same aryl or heteroaryl group.

10. Compounds according to one or more of Claims 1 to 9, **characterised in that** Ar⁷, Ar⁸ and Ar⁹ stand for the same aryl or heteroaryl group.

11. Compounds according to one or more of Claims 1 to 10, **characterised in that** they have a symmetrical structure and a three-fold axis of rotation (C3).

12. Use of compounds according to one or more of Claims 1 to 11 in organic electronic devices.

13. Organic electronic devices comprising at least one compound according to one or more of Claims 1 to 11, selected from the group of the organic electroluminescent devices (OLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs) and organic laser diodes (O-lasers).

14. Organic electroluminescent device comprising anode, cathode and at least one emitting layer, **characterised in that** the emitting layer comprises at least one compound according to one or more of Claims 1 to 11.

15. Organic electroluminescent device according to Claim 14, **characterised in that** the compound according to one or more of Claims 1 to 11 is employed as a mixture with at least one host material.

16. Organic electroluminescent device according to Claim 15, **characterised in that** the host material is selected from the classes of the oligoarylenes, the oligoarylenes containing condensed aromatic groups, the oligoarylenevinylenes, the polypodal metal complexes, the hole-conducting compounds, the electron-conducting compounds, the ketones, the phosphine oxides, the sulfoxides and the atropisomers.

17. Organic electroluminescent device according to Claim 15 and/or 16, **characterised in that** the proportion of the compound according to one or more of Claims 1 to 11 in the mixture is between 0.1 and 99.0% by weight and the proportion of the host material is correspondingly between 1.0 and 99.9% by weight.

18. Organic electroluminescent device according to one or more of Claims 14 to 17, **characterised in that** further layers selected from hole-injection layer, hole-transport layer, electron-transport layer and/or electron-injection layer are also present.

19. Organic electroluminescent device according to one or more of Claims 14 to 18, **characterised in that** a plurality of emitting compounds are used in the same layer or in different layers, where at least one of these compounds has a structure of the formula (1).

## Revendications

1. Composés de formule (1) 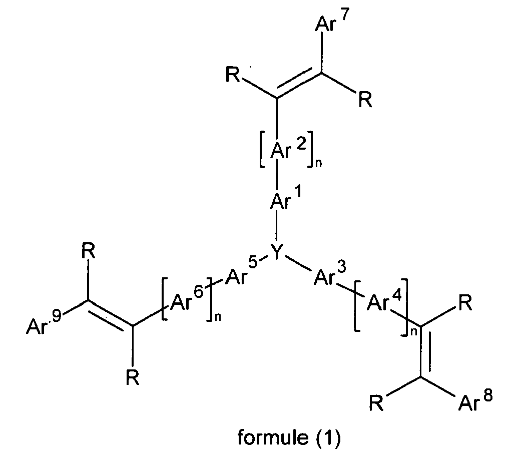 où ce qui suit s'applique aux symboles et indices utilisés :
Y est à chaque occurrence azote, phosphore, arsenic, antimoine, P=O, As=O ou Sb=O;
Ar¹, Ar², Ar³, Ar⁴, Ar⁵, Ar⁶ sont à chaque occurrence, de manière identique ou différente, un groupement aryle ou hétéroaryle divalent ayant de 2 à 24 atomes de C, pouvant être substitué par un ou plusieurs radicaux R¹;
Ar⁷, Ar⁸, Ar⁹ sont à chaque occurrence, de manière identique ou différente, un groupement aryle ou hétéroaryle monovalent ayant de 2 à 24 atomes de C, pouvant être substitué par un ou plusieurs radicaux R¹;
R est à chaque occurrence, de manière identique ou différente, H, CN, un groupement alkyle à chaîne linéaire, ramifiée, ou cyclique ayant de 1 à 40 atomes de C, pouvant être substitué par un ou plusieurs radicaux R², où un ou plusieurs groupements CH₂ non adjacents peuvent être remplacés par -R²C=CR²-, -C≡C-, Si(R²)₂, Ge(R²)₂, Sn(R²)₂, C=O, C=S, C=Se, C=NR², -O-, -S- ou -CONR²- et où un ou plusieurs atomes de H peuvent être remplacés par F, CI, Br, I, CN ou NO₂, ou un groupement aryle ou hétéroaryle mono-valent ayant de 2 à 24 atomes de C, pouvant être substitué par un ou plusieurs radicaux R¹;
R¹ est à chaque occurrence, de manière identique ou différente, H, F, CI, Br, I, CN, NO₂, un groupement alkyle, alcoxy ou thioalcoxy à chaîne linéaire, ramifiée, ou cyclique ayant de 1 à 40 atomes de C, pouvant être substitué par un ou plusieurs radicaux R², où un ou plusieurs groupements CH₂ non adjacents peuvent être remplacés par -R²C=CR²-, -C≡C-, Si(R²)₂, Ge(R²)₂, Sn(R²)₂, C=O, C=S, C=Se, C=NR², -O-, -S- ou -CONR²- et où un ou plusieurs atomes de H peuvent être remplacés par F, CI, Br, I, CN ou NO₂, ou un groupement aryle ou hétéroaryle ayant de 2 à 24 atomes de C, pouvant être substitué par un ou plusieurs radicaux R², ou un groupement aryloxy ou hétéroaryloxy ayant de 2 à 24 atomes de C aromatiques, pouvant être substitué par un ou plusieurs radicaux R², ou une association de deux, trois, quatre ou cinq de ces systèmes ; deux ou plusieurs substituants R¹ ici, tous deux sur le même cycle et aussi sur des cycles différents, peuvent également former un cycle mono- ou polycyclique, aliphatique ou aromatique les uns avec les autres ;
R² est à chaque occurrence, de manière identique ou différente, H ou un radical hydrocarboné aliphatique ou aromatique ayant de 1 à 20 atomes de C ;
n vaut à chaque occurrence, de manière identique ou différente, 1, 2, 3, 4 ou 5.

2. Composés selon la revendication 1, **caractérisés en ce que** le symbole Y représente azote, phosphore ou P=O.

3. Composés selon la revendication 1 et/ou 2, **caractérisés en ce que** les symboles Ar¹, Ar², Ar³, Ar⁴, Ar⁵ et Ar⁶, de manière identique ou différente à chaque occurrence, représentent un groupement aryle ou hétéroaryle divalent ayant de 2 à 16 atomes de C, pouvant être substitué par un ou deux radicaux R¹.

4. Composés selon l'une ou plusieurs des revendications 1 à 3, **caractérisés en ce que** les symboles Ar⁷, Ar⁸ et Ar⁹, de manière identique ou différente à chaque occurrence, représentent un groupement aryle ou hétéroaryle monovalent ayant de 2 à 16 atomes de C, pouvant être substitué par un ou plusieurs radicaux R¹.

5. Composés selon l'une ou plusieurs des revendications 1 à 4, **caractérisés en ce que** le symbole R, de manière identique ou différente à chaque occurrence, représente H, CN, un groupement alkyle à chaîne linéaire ou ramifiée ayant de 1 à 4 atomes de C, où un ou plusieurs groupements CH₂ non adjacents peuvent être remplacés par -R²C=CR²-, -C≡C-, -O- ou -S- et où un ou plusieurs atomes de H peuvent être remplacés par F, ou un groupement aryle ou hétéroaryle monovalent ayant de 2 à 16 atomes de C, pouvant être substitué par un ou plusieurs radicaux R¹.

6. Composés selon l'une ou plusieurs des revendications 1 à 5, **caractérisés en ce que** le symbole R¹, de manière identique ou différente à chaque occurrence, représente H, F, CN, un groupement alkyle ou alcoxy à chaîne linéaire, ramifiée ou cyclique ayant de 1 à 10 atomes de C, pouvant être substitué par un ou plusieurs radicaux R², où un ou plusieurs groupements CH₂ non adjacents peuvent être remplacés par -R²C=CR²-, -C≡C-, Si(R²)₂, C=O, -O- ou -S- et où un ou plusieurs atomes de H peuvent être remplacés par F, ou un groupement aryle ou hétéroaryle ayant de 2 à 16 atomes de C, pouvant être substitué par un ou plusieurs radicaux R², ou un groupement aryloxy ou hétéroaryloxy ayant de 2 à 16 atomes de C aromatiques, pouvant être substitué par un ou plusieurs radicaux R², ou une association de deux, trois ou quatre de ces systèmes ; deux ou plusieurs substituants R¹ ici, tous deux sur le même cycle et aussi sur des cycles différents, peuvent également former un cycle mono- ou polycyclique, aliphatique ou aromatique les uns avec les autres.

7. Composés selon l'une ou plusieurs des revendications 1 à 6, **caractérisés en ce que** l'indice n, de manière identique ou différente à chaque occurrence, représente 1, 2 ou 3.

8. Composés selon l'une ou plusieurs des revendications 1 à 7, **caractérisés en ce que** Ar¹, Ar³ et Ar⁵ représentent le même groupement aryle ou hétéroaryle.

9. Composés selon l'une ou plusieurs des revendications 1 à 8, **caractérisés en ce que** Ar², Ar⁴ et Ar⁶ représentent le même groupement aryle ou hétéroaryle.

10. Composés selon l'une ou plusieurs des revendications 1 à 9, **caractérisés en ce que** Ar⁷, Ar⁸ et Ar⁹ représentent le même groupement aryle ou hétéroaryle.

11. Composés selon l'une ou plusieurs des revendications 1 à 10, **caractérisés en ce qu'**ils possèdent une structure symétrique et un triple axe de rotation (C3).

12. Utilisation de composés selon l'une ou plusieurs des revendications 1 à 11, dans des dispositifs électroniques organiques.

13. Dispositifs électroniques organiques comprenant au moins un composé selon l'une ou plusieurs des revendications 1 à 11, choisis parmi le groupe constitué par les dispositifs électroluminescents organiques (OLED), les transistors organiques à effet de champ (O-FET), les transistors organiques à couches minces (O-TFT), les transistors organiques électroluminescents (O-LET), les circuits intégrés organiques (O-IC), les cellules solaires organiques (O-SC), les dispositifs organiques de coupure de champ (O-FQD) et les diodes laser organiques (O-lasers).

14. Dispositif électroluminescent organique comprenant une anode, une cathode et au moins une couche émettrice, **caractérisé en ce que** la couche émettrice comprend au moins un composé selon l'une ou plusieurs des revendications 1 à 11.

15. Dispositif électroluminescent organique selon la revendication 14, **caractérisé en ce que** le composé selon l'une ou plusieurs des revendications 1 à 11 est employé comme mélange avec au moins un matériau hôte.

16. Dispositif électroluminescent organique selon la revendication 15, **caractérisé en ce que** le matériau hôte est choisi parmi les classes des oligoarylènes, des oligoarylènes contenant des groupements aromatiques condensés, des oligoarylène-vinylènes, des complexes métalliques polypodes, des composés de conduction par trous, des composés de conduction par électrons, des cétones, des oxydes de phosphine, des sulfoxydes et des atropisomères.

17. Dispositif électroluminescent organique selon la revendication 15 et/ou 16, **caractérisé en ce que** la proportion du composé selon l'une ou plusieurs des revendications 1 à 11 dans le mélange se trouve entre 0,1 et 99,0% en poids et la proportion du matériau hôte se trouve de manière correspondante entre 1,0 et 99,9% en poids.

18. Dispositif électroluminescent organique selon l'une ou plusieurs des revendications 14 à 17, **caractérisé en ce que** des couches supplémentaires choisies parmi une couche d'injection de trous, une couche de transport de trous, une couche de transport d'électrons et/ou une couche d'injection d'électrons, sont également présentes.

19. Dispositif électroluminescent organique selon l'une ou plusieurs des revendications 14 à 18, **caractérisé en ce qu'**une pluralité de composés émetteurs est utilisée dans la même couche ou dans des couches différentes, où au moins l'un de ces composés possède une structure de formule (1).
